# EUROPEAN PATENT APPLICATION

(11) **EP 3 588 335 A1**
(43) Date of publication of application: **01.01.2020**
(21) Application number: 17897638.7
(22) Date of filing: 27.02.2017
(51) Int. Cl.: G06F 19/16

(54) **METHOD AND DEVICE FOR CALCULATING STABLE THREE-DIMENSIONAL STRUCTURE, AND PROGRAM**

(71) Applicant: Fujitsu Limited, Kawasaki-shi, Kanagawa 211-8588 (JP)
(72) Inventor: TOMONAGA, Atsushi, Kawasaki-shi Kanagawa 211-8588 (JP); SUGIYAMA, Hajime, Kawasaki-shi Kanagawa 211-8588 (JP); UEDA, Akihiko, Kawasaki-shi Kanagawa 211-8588 (JP)
(74) Representative: Haseltine Lake Kempner LLP
(86) International application number: PCT/JP2017/007565
(87) International publication number: WO 2018/154789

(57) **Abstract**

Provided is a method for calculating stable conformations, the method including: converting stable conformation data of an (n - a)-membered cyclic compound [with the proviso that a is a positive integer and (n - a) ≥ 3] into conformation data of an n-membered cyclic compound (with the proviso that n is an integer of 4 or greater) using data related to an atomic group including atoms in the number of at least a; and minimizing energy of the n-membered cyclic compound using the conformation data of the n-membered cyclic compound, wherein the method is a method for calculating stable conformations of the n-membered cyclic compound using a computer.

## Description

### FIELD

The embodiments discussed herein relate to a method and device for calculating stable conformations of a cyclic compound, and a program for executing the method.

### BACKGROUND

Stable conformations of a compound largely influence characteristics of the compound. Therefore, it is very important to know stable conformations of a compound. In the field of drug discovery, particularly, knowing stable conformations of a compound is very important in determining binding free energy between the compound and protein.

A systematic search method has been known as a method for determining stable conformations of a compound. However, it is very difficult to determine stable conformations of a cyclic compound according to the systematic search method.

As a method for determining stable conformations of a cyclic compound, therefore, the CONFLEX system (see, for example, NPL 1), and the Ring Opening system have been known. These systems have a problem that it takes a long time to complete a calculation. For example, the ring opening system is a system where a ring is cut to form a pseudo-acyclic molecule and the pseudo-acyclic molecule is treated as a regular acyclic molecule. In the ring opening system, however, structures to be generated increase exponentially, as the number of members increases. Therefore, there is a problem that it takes a very long time to complete a calculation.

### Citation List

### Non-Patent Literature

NPL 1: Hitoshi Goto, and Eiji Osawa, J. CHEM. SOC. PERKIN TRANS., 2, (1993)

### SUMMARY

The disclosed embodiments aim to solve the above-described various problems existing in the art, and to achieve the following object. Specifically, the present disclosure has an object to provide a method for calculating stable conformations of a cyclic compound within a short period of time, a device for calculating stable conformations where the device is capable of calculating the stable conformation of a cyclic compound within a short period of time, and a program for executing the method.

Means for solving the above-described problems are as follows.

The disclosed method for calculating stable conformations is a method for calculating stable conformations of an n-membered cyclic compound (with the proviso that n is an integer of 4 or greater) using a computer. The method includes converting stable conformation data of an (n - a)-membered cyclic compound [with the proviso that a is a positive integer and (n - a) ≥ 3] into conformation data of the n-membered cyclic compound (with the proviso that n is an integer of 4 or greater) using data related to an atomic group including atoms in the number of at least a, and minimizing energy of the n-membered cyclic compound using the conformation data of the n-membered cyclic compound.

The disclosed program is a program for causing a computer to execute a method for calculating stable conformations of an n-membered cyclic compound. The method includes converting stable conformation data of an (n - a)-membered cyclic compound [with the proviso that a is a positive integer and (n - a) ≥ 3] into conformation data of an n-membered cyclic compound (with the proviso that n is an integer of 4 or greater) using data related to an atomic group including atoms in the number of at least a, and minimizing energy of the n-membered cyclic compound using the conformation data of the n-membered cyclic compound.

The disclosed device for calculating stable conformations is a device for calculating stable conformations of an n-membered cyclic compound (with the proviso that n is an integer of 4 or greater). The device includes a converting unit configured to convert stable conformation data of an (n - a)-membered cyclic compound [with the proviso that a is a positive integer and (n - a) ≥ 3] into conformation data of the n-membered cyclic compound (with the proviso that n is an integer of 4 or greater) using data related to an atomic group including atoms in the number of at least a, and a minimizing unit configured to minimize energy of the n-membered cyclic compound using the conformation data of the n-membered cyclic compound.

The disclosed method for calculating stable conformations can determine a stable conformation of a cyclic compound within a short period of time.

The disclosed program can determine a stable conformation of a cyclic compound within a short period of time.

The disclosed device for calculating stable conformations can determine a stable conformation of a cyclic compound within a short period of time.

### BRIEF DESCRIPTION OF DRAWINGS

FIG. 1A is a schematic view illustrating part of a cyclic structure of an (n - 1)-membered cyclic compound.
FIG. 1B is a schematic view illustrating a state where the bond B1 is eliminated in FIG. 1A.
FIG. 1C is a view illustrating a state where an atom A3 is inserted in FIG. 1B.
FIG. 1D is a schematic view illustrating part of the cyclic structure of the n-membered ring.
FIG. 2A is a schematic view illustrating part of a cyclic structure of an (n - 1)-membered cyclic compound.
FIG. 2B is a schematic view illustrating a state where the bond B1 is eliminated in FIG. 2A.
FIG. 2C is a view illustrating a state where an atom A3 is inserted in FIG. 2B.
FIG. 2D is a schematic view illustrating part of the cyclic structure of the n-membered ring.
FIG. 3A is a schematic view illustrating part of a cyclic structure of an (n - 2)-membered cyclic compound.
FIG. 3B is a schematic view illustrating a state where the bond B1 is eliminated in FIG. 3A.
FIG. 3C is a schematic view illustrating a state where an atom A4 and an atom A5 are inserted.
FIG. 3D is a schematic view illustrating part of the cyclic structure of the n-membered cyclic compound.
FIG. 4 is a flowchart illustrating one example of the disclosed method for calculating stable conformations.
FIG. 5 is a flowchart illustrating another example of the disclosed method for calculating stable conformations.
FIG. 6 is a flowchart illustrating another example of the disclosed method for calculating stable conformations.
FIG. 7 is a view illustrating a structural example of the disclosed device for calculating stable conformations.
FIG. 8 is a view illustrating another structural example of the disclosed device for calculating stable conformation.
FIG. 9 is a view illustrating another structural example of the disclosed device for calculating stable conformations.

### DESCRIPTION OF EMBODIMENTS

Drug discovery refers to a process for designing pharmaceutical products. For example, the drug discovery is performed in the following order.
(1) Determination of a target molecule
(2) Searching a lead compound etc.
(3) Examination of physiological effects
(4) Safety/toxicity test

It is important in the search of a lead compound etc. (a lead compound and a compound derived from the lead compound) that interaction between each of numerous drug candidate molecules and a target molecule is highly accurately evaluated.

A process for designing pharmaceutical products using a computer can be used for drug discovery in general. Among them, use of the IT drug discovery in a search of a lead compound etc. is effective for reducing a time period for and increasing a probability of developing a new drug.

For example, the disclosed technology can be used for a search of a lead compound that is expected to have high pharmacological activity.

In addition to the drug discovery, moreover, the disclosed technology can be used for a research of physical properties of a cyclic compound. The cyclic compound may be a known compound or unknown compound.

### (Method for calculating stable conformation)

The disclosed method for calculating stable conformations is a method for calculating stable conformations of an n-membered cyclic compound (with the proviso that n is an integer of 4 or greater) using a computer.

Known methods for calculating stable conformations of cyclic compounds have a problem that a calculation takes a long time to complete. In the ring opening system, for example, structures to be generated increase exponentially, as the number of members increases. Therefore, it takes a very long time to complete a calculation.

Therefore, the present inventors have found the following insights. That is, when stable conformations of an n-membered cyclic compound (n is an integer of 4 or greater) are determined, a calculation time can be shortened by determining stable conformations of the n-membered cyclic structure based on stable conformations of a cyclic compound whose number of members is less than that of the n-membered ring [(n - a)-membered cyclic compound, with the proviso that a is a positive integer and (n - a) ≥ 3]. The disclosed invention has been accomplished based on the above-described insights.

When stable conformations of a cyclic compound are considered, local structural stability of a site where an atom is inserted significantly changes as the atom is inserted to a ring structure to increase the number of members, but local structural stability of a site far from the inserted site does not largely change. When stable conformations of an n-membered ring are determined according to the above-described method, stable conformations can be efficiently calculated even in the case where the number of conformations on which energy minimization is performed is small.

Specifically, the method for calculating stable conformations includes converting stable conformation data of an (n - a)-membered cyclic compound [with the proviso that a is a positive integer and (n - a) ≥ 3] into conformation data of the n-membered cyclic compound using data related to an atomic group including atoms in the number of at least a.

Moreover, the method for calculating stable conformations includes minimizing energy of the n-membered cyclic compound using the conformation data of the n-membered cyclic compound.

In the present specification, the term "stable conformation data" includes, for example, atom information data, coordinate information data, and bond information data, and creates stable conformations in a coordinate space.

In the present specification, the term "conformation date" includes, for example, atom information data, coordinate information data, and bond information data, and creates conformations in a coordinate space.

In the present specification, the term "data related to an atomic group" includes, for example, atom information data, coordinate information data, and bond information data, and creates conformations in a coordinate space.

Formats of the above-mentioned sets of data are not particularly limited and may be appropriately selected depending on the intended purpose. For example, a format of data may be text data, or a structure data file (SDF) format, or a MOL file format.

The atom information data is data related to a type of an atom.

The coordinate information data is data related to coordinates (a position) of an atom.

The bond information data is data related to a bond between an atom and an atom.

### <Converting into conformation data of n-membered cyclic compound>

The method for calculating stable conformations includes converting stable conformation data of an (n - a)-membered cyclic compound [with the proviso that a is a positive integer and (n - a) ≥ 3] into conformation data of the n-membered cyclic compound. The converting is performed using data related to an atomic group including atoms in the number of at least a. For example, the converting is performed by inserting the atomic group including atoms in the number of at least a adjacent to a ring of the (n - a) membered cyclic compound.

The term "adjacent" means a range where the atoms in the number of at least a are capable of forming bonds with atoms constituting the ring of the (n - a)-membered cyclic compound. A distance capable of forming the bonds may vary depending on a type of a bond generated or an environment surrounding atoms to be bonded. The distance is not particularly limited and may be appropriately selected depending on the intended purpose. The distance may be selected by referring to known bonding distances.

The stable conformation data of the (n - a)-membered cyclic compound (with the proviso that a is a positive integer and n > a) may be known data, or data determined by a known method, or data determined by the disclosed method for calculating.

The stable conformation data may not be the most stable conformation data of a target molecule. For example, the stable conformation data may be stable conformation data determined by a known method, such as energy minimization. Therefore, the stable conformation data of the (n - a)-membered cyclic compound may be single data, or a set of data, but the stable conformation data thereof is typically a set of data.

The cyclic compound may be a monocyclic compound or a polycyclic compound.

A type of a ring in the cyclic compound is not particularly limited and may be appropriately selected depending on the intended purpose. For example, the ring may be a hydrocarbon ring, or an aromatic ring, or a heterocycle.

The atomic group includes atoms in the number of at least a. The atoms in the number of at least a are inserted into a cyclic structure of the (n - a)-membered cyclic compound.

The number a is not particularly limited as long as the number a is a positive integer and may be appropriately selected depending on the intended purpose. The number a is preferably from 1 to 10, and more preferably from 1 to 5. When the number a is too large, a difference between the number of members of the (n - a)-membered cyclic compound and the number of members of the n-membered cyclic compound becomes too large and therefore stable conformations thereof are largely different from each other. As a result, effective stable conformations of the n-membered cyclic compound may not be obtained.

The number of atoms of the atomic group is not particularly limited and may be appropriately selected depending on the intended purpose. The number of atoms thereof is preferably from 1 to 100, more preferably from 1 to 50, and particularly preferably from 1 to 30. Namely, the number of atom(s) of the atomic group may be one.

The atoms of the inserted atomic group, where the number of the atoms is a, may be arranged on a straight line connecting arbitrary two atoms constituting a bond constituting a ring of the (n - a)-membered cyclic compound, or may be arranged on positions other than on the straight chain.

In the course of the converting, for example, the atomic group is inserted between two atoms constituting a bond constituting a ring of the (n - a)-membered cyclic compound.

In the present specification, the phrase "between two atoms" does not limit to on a straight line connecting between two atoms. The phrase "between two atoms" means a space between a first plane perpendicular to a straight line connecting the two atoms and a second plane perpendicular to the straight line connecting the two atoms. Moreover, the case where the atoms of the atomic group, where the number of the atoms is a, are present between the two planes is regarded as that the atomic group is inserted "between two atoms" even though all of the atoms of the atomic group are not present between the two planes.

The bond formed between the two atoms, to which the atomic group is inserted, is not particularly limited and may be appropriately selected depending on the intended purpose. Examples of the bond include a single bond, a double bond, and a triple bond. Moreover, examples thereof include a carbon-carbon bond, a carbon-oxygen bond, a carbon-nitrogen bond, and a carbon-sulfur bond.

When the atomic group is inserted, the coordinates of the two atoms may be changed or may not be changed.

The converting into conformation data includes, for example, converting data related to a bond constituting a ring of the (n - a)-membered cyclic compound into data related to bonds, the number of which is a + 1, generated between two atoms constituting the bond and the atoms in the atomic group where the number of the atoms is a.

The data related to the bond is data included in the steric stable structure data.

A method for performing the above-described converting is not particularly limited and may be appropriately selected depending on the intended purpose. For example, the converting may be performed by changing the stable confirmation data itself. Moreover, the converting may be performed by converting the stable conformation data into a chemical structural diagram using software for drawing a chemical structural formula, inserting an atomic group including atoms in the number of at least a in the chemical structural diagram on the software, and output the converted chemical structural diagram as stable conformation data of the n-membered cyclic compound.

During the converting into the conformation data, a plurality of sets of the conformation data of a single molecule are preferably obtained using the data related to the atomic group as a plurality of coordinate patterns. As a result, a probability of determining stable conformations that are more stable increases.

As one example of "using the data related to the atomic group as a plurality of coordinate patterns," for example, listed is to perform the converting with an identical conformation of the atomic group, but using a plurality of coordinates for arranging the atomic group relative to the stable conformation.

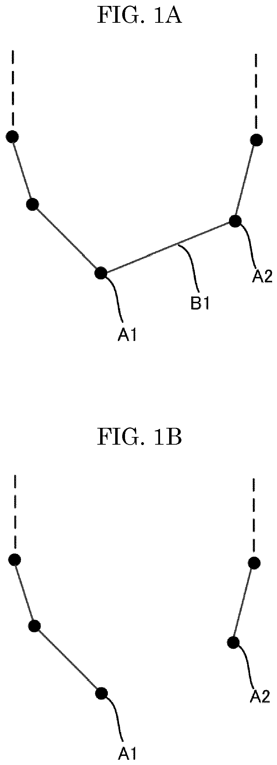

One example of the method for converting the stable conformation data of the (n - 1)-membered cyclic compound into conformation data of the n-membered compound will be explained with reference to FIGs. 1A to 1D.

FIG. 1A is a schematic view illustrating part of a cyclic structure of the (n - 1)-membered cyclic compound.

FIG. 1B is a schematic view illustrating a state where the bond B1 is eliminated in FIG. 1A.

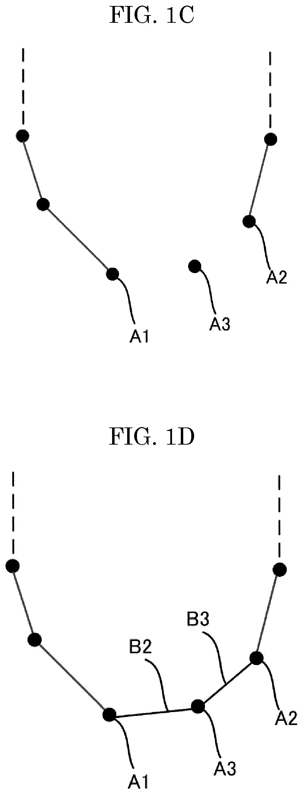

FIG. 1C is a view illustrating a state where an atom A3 is inserted in FIG. 1B.

FIG. 1D is a schematic view illustrating part of the cyclic structure of the n-membered cyclic compound.

In FIGs. 1A to 1D, the solid line represents a bond, the black dot represents an atom, and the broken line represents that a structure is omitted. Note that, illustration of atoms that are bonded to the atoms constituting the cyclic structure but not contributes to the constitution of the cyclic structure is omitted.

In this example, an atomic group including one (a = 1) atom A3 is inserted between two atoms (an atom A1 and an atom A2) constituting a bond B1 constituting a ring to convert stable conformation data of an (n - 1)-membered cyclic compound into conformation data of an n-membered cyclic compound.

Specifically, the conversion is performed in the following manner.

First, bond information data related in the bond B1 in the stable conformation data of the (n - 1)-membered cyclic compound is deleted (FIG. 1B).

Subsequently, atom information data and coordinate data of the atomic group including one (a = 1) atom A3 are added to the stable conformation data (FIG. 1C). During the process as mentioned, coordinates of the atom A3 are determined, for example, in a manner that a distance between the atom A1 and the atom A3 and a distance between the atom A2 and the atom A3 are each shorter than a distance between the atom A1 and the atom A2.

Subsequently, bond information data related to a bond B2 newly generated between the atom A1 and the atom A3 and bond information data related to a bond B3 newly generated between the atom A2 and the atom A3 are added to the stable conformation data (FIG. 1D).

As a result, the stable conformation data of the (n - 1)-membered cyclic compound is converted into conformation data of the n-membered cyclic compound.

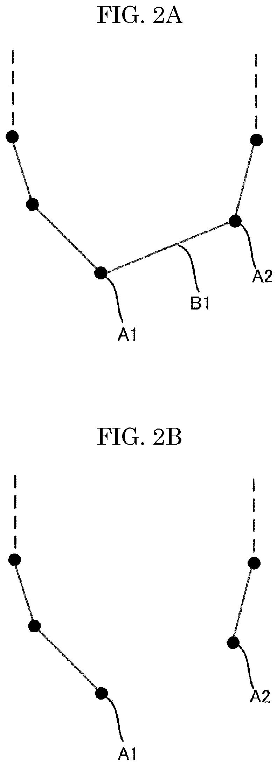

Next, another example of the method for converting the stable conformation data of the (n - 1)-membered cyclic compound into conformation data of the n-membered compound will be described with reference to FIGs. 2A to 2D. This example is an example where coordinates (a position) to which the atom A3 are changed in the example of FIGs. 1A to 1D.

FIG. 2A is a schematic view illustrating part of a cyclic structure of the (n - 1)-membered cyclic compound.

FIG. 2B is a schematic view illustrating a state where the bond B1 is eliminated in FIG. 2A.

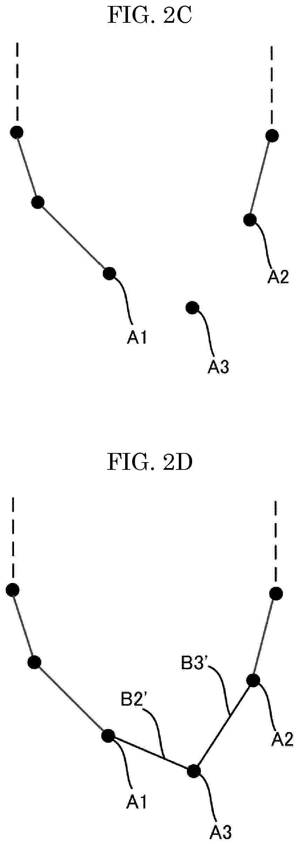

FIG. 2C is a schematic view illustrating a state where an atom A3 is inserted in FIG. 2B.

FIG. 2D is a schematic view illustrating a state where part of the cyclic structure of the n-membered cyclic compound.

In FIGs. 2A to 2D, the solid line represents a bond, the black dot represents an atom, and the broken line represents that a structure is omitted. Note that, illustration of atoms that are bonded to the atoms constituting the cyclic structure but not contributes to the constitution of the cyclic structure is omitted.

In this example, an atomic group including one (a = 1) atom A3 is inserted between two atoms (an atom A1 and an atom A2) constituting a bond B1 constituting a ring to convert stable conformation data of an (n - 1)-membered cyclic compound into conformation data of an n-membered cyclic compound.

Specifically, the conversion is performed in the following manner.

First, bond information data related to the bond B1 in the stable conformation data of the (n - 1)-membered cyclic compound is deleted (FIG. 2B).

Subsequently, atom information data and coordinate data of the atomic group including one (a = 1) atom A3 are added to the stable conformation data (FIG. 2C). During the process as mentioned, coordinates of the atom A3 are determined, for example, in a manner that a distance between the atom A1 and the atom A3 and a distance between the atom A2 and the atom A3 are each shorter than a distance between the atom A1 and the atom A2.

Subsequently, bond information data related to a bond B2' newly generated between the atom A1 and the atom A3 and bond information data related to a bond B3' newly generated between the atom A2 and the atom A3 are added to the stable conformation data (FIG. 2D).

As a result, the stable conformation data of the (n - 1)-membered cyclic compound is converted into conformation data of the n-membered cyclic compound.

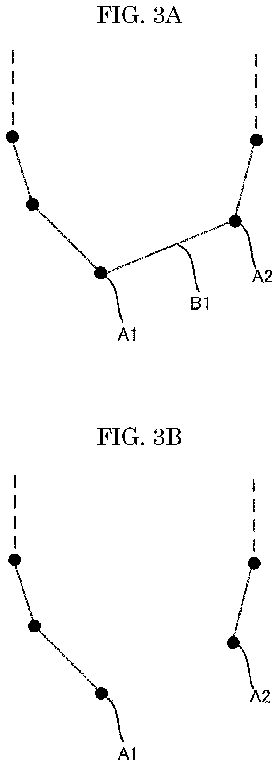

Next, one example of the method for converting the stable conformation data of the (n - 2)-membered cyclic compound into conformation data of the n-membered compound will be described with reference to FIGs. 3A to 3D.

FIG. 3A is a schematic view illustrating part of a cyclic structure of an (n - 2)-membered cyclic compound.

FIG. 3B is a schematic view illustrating a state where the bond B1 is eliminated in FIG. 3A.

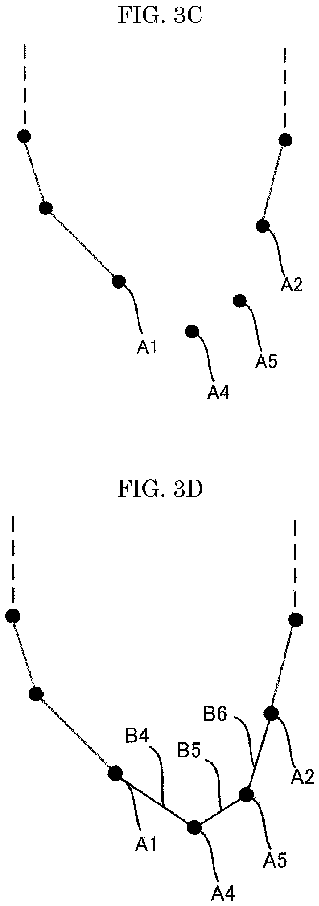

FIG. 3C is a schematic view illustrating a state where an atom A4 and an atom A5 are inserted in FIG. 3B.

FIG. 3D is a schematic view illustrating part of the cyclic structure of the n-membered cyclic compound.

In FIGs. 3A to 3D, the solid line represents a bond, the black dot represents an atom, and the broken line represents that a structure is omitted. Note that, illustration of atoms that are bonded to the atoms constituting the cyclic structure but not contributes to the constitution of the cyclic structure is omitted.

In this example, an atomic group including two (a = 2) atoms A4 and A5 is inserted between two atoms (an atom A1 and an atom A2) constituting a bond B1 constituting a ring to convert stable conformation data of the (n - 2)-membered cyclic compound into conformation data of an n-membered cyclic compound.

Specifically, the conversion is performed in the following manner.

First, bond information data related to the bond B1 in the stable conformation data of the (n - 2)-membered cyclic compound is deleted (FIG. 3B).

Subsequently, atom information data and coordinate data of the atomic group including two atoms A4 and A5 are added to the stable conformation data (FIG. 3C). During the process as mentioned, coordinates of the atoms A4 and A5 are determined in a manner that a distance between the atom A1 and the atom A4, and a distance between the atom A2 and the atom A5 are each shorter than a distance between the atom A1 and the atom A2.

Subsequently, bond information data related to a bond B4 newly generated between the atom A1 and the atom A4, bond information data related to a bond B5 newly generated between the atom A4 and the atom A5, and bond information data related to a bond B6 newly generated between the atom A2 and the atom A5 are added to the stable conformation data (FIG. 3D).

As a result, the stable conformation data of the (n - 2)-membered cyclic compound is converted into conformation data of the n-membered cyclic compound as illustrated in FIG. 3D.

### <Minimizing energy>

The method for calculating stable conformations includes minimizing energy of the n-membered cyclic compound using the conformation data.

The minimizing energy is performed, for example, by molecular dynamics calculations.

For example, the minimizing energy is performed by determining force working on each of atoms of the n-membered cyclic compound and sifting the atoms in the direction to reduce energy.

Examples of the force working on the atom include Coulomb force and Van der Waals force.

The energy minimization calculation is performed, for example, using known algorithms. Examples of the known algorithms include Steepest descent method, Steepest descent method with constraints, and Conjugate gradient method.

The steepest descent method is a method for shifting to the energy minimum using a first derivation (i.e., force) of function of potential energy, which is numerically calculated.

One example of a procedure of a calculation of the steepest descent method will be described below.
- Potential energy and force of the initial structure are calculated.
- One of constitutional atoms thereof is gradually sifted along axial directions in the coordinate system, and energy and force are calculated again every time the atom is sifted.
- The process above is repeated and performed on all of the atoms, and all of the atoms are shifted to new positions along the lower slope direction on the potential energy surface.
- The operation is stopped when the predetermined judgement conditions are satisfied.

According to the above-mentioned algorithm, local minimum on the potential energy surface can be found, but global minimum may not be always found.

The minimizing energy can be performed, for example, using known software.

Examples of a program used for the molecular dynamics calculation include tinker, amber (Assisted Model Building with Energy Refinement), gromacs (Groningen Machine for Chemical Simulations), charm (Chemistry at HARvard Macromolecular Mechanics), and lammps.

The method for calculating stable conformations can be performed using a computer. The computer used in the method for calculating stable conformations may be one computer, or a plurality of computers. For example, the method for calculating stable conformations may be divided and executed by a plurality of computers.

For example, the method for calculating stable conformations can be performed by means of a general computer system (e.g., various network servers, work stations, and personal computers) equipped with a central processing unit (CPU), random access memory (RAM), a hard disk, various peripherals, etc.

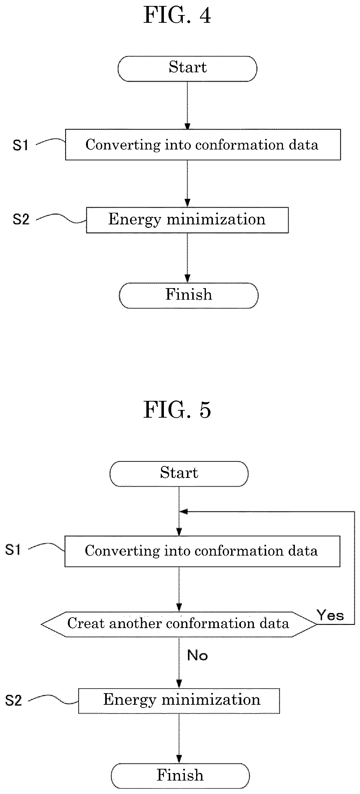

A flowchart of one example of the method for calculating stable conformations is illustrated in FIG. 4.

The flowchart of FIG. 4 illustrates one example for creating one set of conformation data.

First, stable conformation data of an (n - a)-membered [with the proviso that a is a positive integer and (n - a) ≥ 3] cyclic compound is converted to conformation data of an n-membered cyclic compound by inserting an atomic group including atoms in the number of at least a between two atoms constituting a bond constituting a ring of the (n - a)-membered cyclic compound (S1).

Subsequently, energy minimization of the n-membered cyclic compound is performed using the obtained conformation data (S2).

Next, a flowchart of another example of the method for calculating stable conformations is illustrated in FIG. 5.

The flowchart of FIG. 5 illustrates one example for creating a plurality of sets of conformation data.

First, stable conformation data of an (n - a)-membered [with the proviso that a is a positive integer and (n - a) ≥ 3] cyclic compound is converted to conformation data of an n-membered cyclic compound by inserting an atomic group including atoms in the number of at least a between two atoms constituting a bond constituting a ring of the (n - a)-membered cyclic compound (S1).

Next, in the case where another conformation data is created, a step for converting to conformation data (S1) is performed again. In this step, for example, another conformation data of the same molecule in which a position for inserting the atomic group between the two atoms is changed is created. A plurality of sets of conformation data of the same molecule can be obtained by changing a position for inserting the atomic group.

Next, in the case where another conformation data is not further created, energy minimization is performed on each of the obtained sets of conformation data (S2).

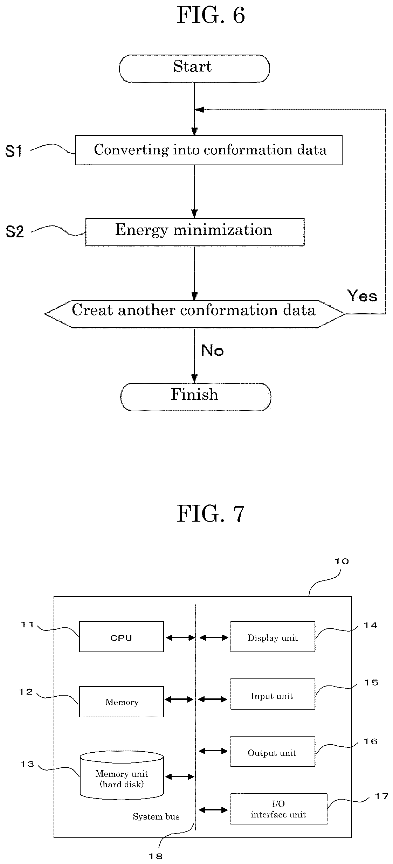

Next, a flowchart of another example of the method for calculating stable conformations is illustrated in FIG. 6.

The flowchart of FIG. 6 illustrates one example for creating a plurality of sets of conformation data.

First, stable conformation data of an (n - a)-membered [with the proviso that a is a positive integer and (n - a) ≥ 3] cyclic compound is converted to conformation data of an n-membered cyclic compound by inserting an atomic group including atoms in the number of at least a between two atoms constituting a bond constituting a ring of the (n - a)-membered cyclic compound (S1).

Next, energy minimization is performed on the obtained conformation data (S2).

Next, in the case where another conformation data is created, a step for converting to conformation data (S1) is performed again. In this step, for example, another conformation data of the same molecule in which a position for inserting the atomic group between the two atoms is changed is created. A plurality of sets of conformation data of the same molecule can be obtained by changing a position for inserting the atomic group.

Next, energy minimization is performed on the obtained conformation data (S2).

In the case where another conformation is not further created, a calculation of stable conformations is finished.

### (Program)

The disclosed program is a program for executing the disclosed method for calculating stable conformations.

The program can be created using any of various programing languages known in the art according to a configuration of a computer system for use, a type or version of an operation system for use.

The program may be recorded on storage media, such as an integral hard disk, and an external hard disk, or recorded on a storage medium, such as a compact disc read only memory (CD-ROM), a digital versatile disk read only memory (DVD-ROM), a magneto-optical (MO) disk, and a universal serial bus (USB) memory stick (USB flash drive). In the case where the program is recorded on a storage medium, such as a CD-ROM, a DVD-ROM, an MO disk, and an USB memory stick, the program can be used, as required, directly or by installing a hard disk via a storage medium reader equipped in a computer system. Moreover, the program may be recorded in an external memory region (e.g. another computer) accessible from the computer system via an information and communication network, and the program may be used, as required, by directly from the external memory region or installing into a hard disk from the external memory region via the information and communication network.

### (Computer-readable non-transitory recording medium)

The disclosed computer-readable non-transitory recording medium has stored therein the disclosed program.

The computer-readable non-transitory recording medium is not particularly limited and may be appropriately selected depending on the intended purpose. Examples of the computer-readable non-transitory recording medium include integral hard disks, external hard disks, CD-ROMs, DVD-ROMs, MO disks, and USB memory sticks.

### (Device for calculating stable conformations)

The disclosed device for calculating stable conformations includes at least a converting unit, and a minimizing unit, and may further include other units according to the necessity.

The converting unit is configured to convert stable conformation data of an (n - a)-membered [with the proviso that a is a positive integer and (n - a) ≥ 3] into conformation data of the n-membered cyclic compound using data related to an atomic group including atoms in the number of at least a.

Moreover, the converting unit is, for example, configured to convert data related to a bond constituting the (n - a)-membered cyclic compound to data relative to bonds, the number of which is a + 1, generated between two atoms constituting the bond and the atoms in the atomic group where the number of the atoms is a.

Moreover, the converting unit is, for example, configured to obtain a plurality of sets of the conformation data of a single molecule using the data related to the atomic group as a plurality of coordinate patterns.

The minimizing unit is configured to minimize energy of the n-membered cyclic compound using the conformation data.

The disclosed device for calculating stable conformations is configured to perform the disclosed method for calculating stable conformations.

A structural example of the disclosed device for calculating stable conformations is illustrated in FIG. 7.

For example, the device for calculating stable conformations 10 includes a CPU 11, a memory 12, a memory unit 13, a display unit 14, an input unit 15, an output unit 16, and an I/O interface unit 17, which are connected via a system bus 18.

The central processing unit (CPU) 11 is configured to perform calculations (e.g., four arithmetic operations, and relational operations), and control of operations of hardware and software. For example, the CPU is an equivalent of the converting unit and the minimizing unit.

The memory 12 is a memory, such as a random access memory (RAM), and a read only memory (ROM). The RAM is configured to store an operating system (OS) and application programs read from the ROM and the memory unit 13, and function as a main memory and work area of the CPU 11.

The memory unit 13 is a device for storing various programs and data. For example, the memory unit 13 is a hard disk. For example, the stable conformation data of the (n-a)-membered ring is stored in the memory unit 13. In the memory unit 13, moreover, programs to be executed by the CPU 11, data for executing the programs, and an OS are stored.

The program is stored in the memory unit 13, loaded on the RAM (a main memory) of the memory 12, and executed by the CPU 11.

The display unit 14 is a display device. For example, the display unit is a display device, such as a CRT monitor, and a liquid crystal panel.

The input unit 15 is an input device for various types of data. Examples of the input unit include a key board, and a pointing device (e.g., a mouse).

The output unit 16 is an output device for various types of data. For example, the output unit is a printer.

The I/O interface unit 17 is an interface for connecting to various external devices. For example, the I/O interface unit enables input and output of data of CD-ROMs, DVD-ROMs, MO disks, and USB memory sticks.

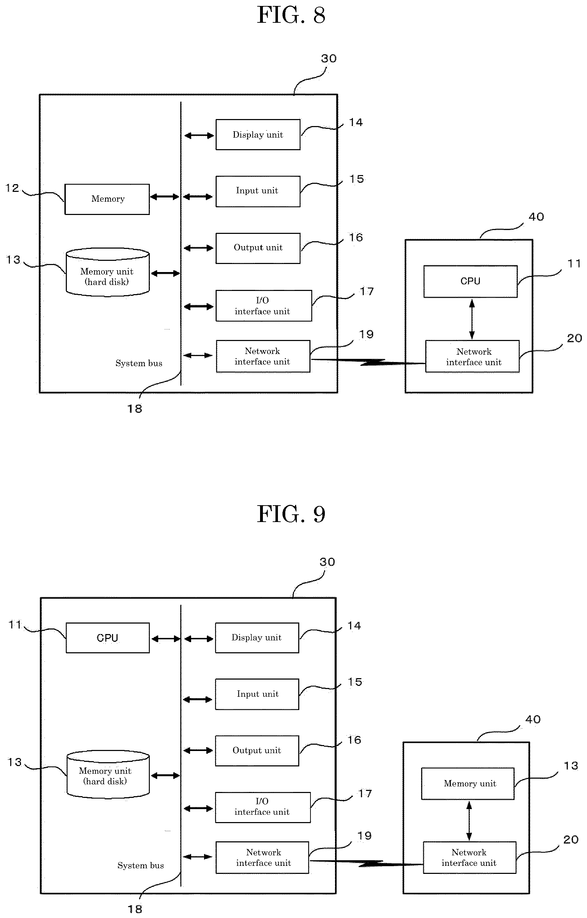

FIG. 8 illustrates another structural example of the disclosed device for calculating stable conformations.

The structural example of FIG. 8 is a structural example of a cloud-type calculation device, where CPU 11 is independent of a memory unit 13. In the structural example, a computer 30 having stored therein the memory device 13 etc., and a computer 40 having stored therein the CPU 11 are coupled with each other via network interface units 19 and 20.

The network interface units 19 and 20 are hardware configured to communicate using Internet.

FIG. 9 illustrates another structural example of the disclosed device for calculating stable conformations.

The structural example of FIG. 9 is a structural example of a cloud-type calculation device, where a memory unit 13 is independent of CPU 11. In the structural example, the CPU 11 is stored therein via network interface units 19 and 20.

### Examples

The disclosed technology will be described hereinafter, but Examples below shall not be construed as to limit the scope of the disclosed technology. Note that, a method for converting data is not specifically described below. However, conversion of a stable conformation of an (n - a)-membered ring to conformation of an n-membered ring was performed by converting stable conformation data of the (n - a)-membered ring into confirmation data of the n-membered ring.

### (Example 1)

### <7-Membered cyclic hydrocarbon>

Stable conformations of 7-membered cyclic hydrocarbon (cycloheptane) were determined by the disclosed technology.

A methylene group was inserted in each of the following 4 bonds in each of two stable conformations (chair and boat) of 6-membered cyclic hydrocarbon (cyclohexane). In each of the structures below, a bond to which insertion is to be performed is presented with a bold line.

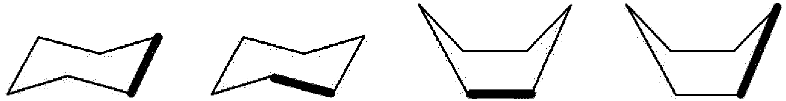

As a result, 4 initial structures represented by the structures below were obtained.

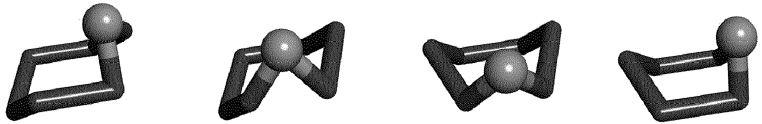

Energy minimization was performed on the four initial structures above to obtain stable conformations. The time spent on the four calculations of the energy minimization was 45 seconds.

Note that, the following calculator was used for the calculations of the energy minimization.

### • PRIMERGY RX200 S6 , Xeon(R) X5650, 24 GB memory

Only one core was used without performing parallel computation.

### (Comparative Example 1)

### <7-Membered cyclic hydrocarbon>

Stable conformations of 7-membered cyclic hydrocarbon (cycloheptane) were determined according to the ring opening method.

One carbon-carbon bond of 7-membered cyclic hydrocarbon (cycloheptane) having the structure below was cut to open a ring, and dihedral angle of the carbon-carbon bond was changed per 90 degrees. The same operation was performed on 6 carbon-carbon bonds to generate 4⁵ = 1,024 structures in total.

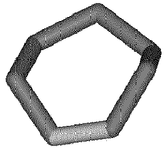

Out of the 1,024 structures, 109 structures satisfying the closed ring conditions (a distance between a pair of atoms causing ring opening was within 5 Å) were selected. Energy minimization was performed on the 109 structures using the calculator used in Example 1 to obtain a plurality of stable conformations the energy values of which were within 5 kcal/mol from the minimum. The time spent on the 109 calculations of the energy minimization was 95 seconds.

Note that the same stable conformations were obtained both in Example 1 and in Comparative Example 1.

### (Example 2)

### <10-Membered cyclic hydrocarbon>

Stable conformations of 7-membered cyclic hydrocarbon (cycloheptane) were determined using 6-membered cyclic hydrocarbon (cyclohexane) in the same manner as in Example 1.

Stable conformations of 8-membered cyclic hydrocarbon (cyclooctane) were determined using the determined stable conformations of 7-membered cyclic hydrocarbon (cycloheptane) in the same manner as in Example 1.

Moreover, stable conformations of 9-membered cyclic hydrocarbon (cyclononane) were determined using the determined stable conformations of 8-membered cyclic hydrocarbon (cyclooctane) in the same manner as in Example 1.

Furthermore, stable conformations of 10-membered cyclic hydrocarbon (cyclodecane) were determined using the determined stable conformations of 9-membered cyclic hydrocarbon (cyclononane) in the same manner as in Example 1.

As a result, the time spent on the energy minimization calculation of the above-mentioned conformations was 354 seconds.

### (Comparative Example 2)

### <10-Membered cyclic hydrocarbon>

Stable conformations were determined in the same manner as in Comparative Example 1, except that the 7-membered cyclic hydrocarbon (cycloheptane) was changed to 10-membered cyclic hydrocarbon (cyclodecane).

As a result, the time spend on calculations of the energy minimization of the conformations was 1,326 seconds.

### (Example 3)

### <11-Membered cyclic hydrocarbon>

Stable conformations of 7-membered hydrocarbon (cycloheptane) were determined using 6-membered hydrocarbon (cyclohexane) in the same manner as in Example 1.

Moreover, stable conformations of 8-membered cyclic hydrocarbon (cyclooctane) were determined using the determined stable conformations of 7-membered cyclic hydrocarbon (cycloheptane) in the same manner as in Example 1.

Moreover, stable conformations of 9-membered cyclic hydrocarbon (cyclononane) were determined using the determined stable conformations of 8-membered cyclic hydrocarbon (cyclooctane) in the same manner as in Example 1.

Moreover, stable conformations of 10-membered cyclic hydrocarbon (cyclodecane) were determined using the determined stable conformations of 9-membered cyclic hydrocarbon (cyclononane) in the same manner as in Example 1.

Furthermore, stable conformations of 11-membered cyclic hydrocarbon (cycloundecane) were determined using the determined stable conformations of 10-membered cyclic hydrocarbon (cyclodecane) in the same manner as in Example 1.

As a result, the time spent on calculations of energy minimization of the conformations was 866 seconds.

### (Comparative Example 3)

### <11-Membered cyclic hydrocarbon>

Stable conformations were determined in the same manner as in Comparative Example 1, except that the 7-membered cyclic hydrocarbon (cycloheptane) was changed to 11-membered cyclic hydrocarbon (cycloundecane).

As a result, the time spent on calculations of energy minimization of the conformations was 3,719 seconds.

The calculation time of Examples 1 to 3 and Comparative Examples 1 to 3 was summarized in Table 1.

**Table 1**

| | Comparative Examples 1 to 3 (sec) | Examples 1 to 3 (sec) |
|---|---|---|
| 7-membered ring | 95 | 45 |
| 10-membered ring | 1,326 | 354 |
| 11-membered ring | 3,719 | 866 |

### (Example 4)

### <Cyclic peptide>

Stable conformations of cyclic peptide in which 6 glycines were bonded to form a ring were determined by the disclosed technology.

First, conformations of cyclic peptide in which 5 glycines were bonded to form a ring were formed according to the ring-opening method. As a result, 310 conformations were obtained.

Subsequently, a glycine residue was inserted into each of 5 amide bonds of 5 cyclic peptide residues to create 310×5 = 1,550 conformations in total. Energy minimization was performed on the conformations. Note that, energy minimization was performed on 1,073 conformations excluding structures where an amide bond was a cis amide bond and duplicated structures.

In the same manner as described above, cyclic peptide in which 7 glycines were bonded to form a ring was obtained. The number of conformations thereof was 6,084. Energy minimization was performed on the conformations.

In the same manner, cyclic peptide in which 8 glycines were bonded to form a ring was obtained. The number of confirmations thereof was 16,684. Energy minimization was performed on these conformations.

Next, out of the conformations above, low energy structures energy of which was within 15 kcal/mol from the minimum energy were selected and used as input structure for the following structure generation.

Out of the 16,684 conformations of the cyclic peptide in which 8 glycines were bonded to form a ring, 6,543 conformations satisfying the above-described conditions were selected. Insertion of glycine was further performed to obtain 23,462 conformations of cyclic peptide in which 9 glycines were bonded to form a ring. Energy minimization was performed on the conformations.

Out of the 23,462 conformations of cyclic peptide in which 9 glycines were bonded to form a ring, furthermore, 20,056 conformations satisfying the above-described conditions were selected. Insertion of glycine was further performed to obtain 67,046 conformations of cyclic peptide in which 10 glycines were bonded to form a ring. Energy minimization was performed on the conformations.

The time spent on the above-described calculations is as presented in Table 2.

Note that, in Table 2, the time in the column of "6 residues" is a time spent on energy minimization of a plurality of conformations of cyclic peptide in which 6 glycines are bonded to form a ring.

In Table 2, the time in the column of "8 residues" is a time spent on energy minimization of a plurality of conformations of cyclic peptide in which 6 glycines are bonded to form a ring, a plurality of conformations of cyclic peptide in which 7 glycines are bonded to form a ring, and a plurality of conformations of cyclic peptide in which 8 glycines are bonded to form a ring.

In Table 2, the time in the column of "10 residues" is a time spent on energy minimization of a plurality of conformations of cyclic peptide in which 6 glycines are bonded to form a ring, a plurality of conformations of cyclic peptide in which 7 glycines are bonded to form a ring, a plurality of conformations of cyclic peptide in which 8 glycines are bonded to form a ring, a plurality of conformations of cyclic peptide in which 9 glycines are bonded to form a ring, and a plurality of conformations of cyclic peptide in which 10 glycines are bonded to form a ring.

Note that, the following calculator was used for the calculations of energy minimization.

### • CELCIUS W510, Intel(R) Xeon(R) CPU E3128, 16 GB memory

Only one core was used without performing parallel computation.

### (Comparative Example 4)

Conformations of cyclic peptide in which 6 glycines were bonded to form a ring and cyclic peptide in which 8 glycines were bonded to form a ring were determined in accordance with the ring opening method, and then energy minimization was performed. Note that, in the same manner as in Comparative Example 1, a plurality of conformations of one bond formed by ring opening were produced by changing a dihedral angle per 90 degrees. Out of the produced conformations, conformations satisfying the closed ring conditions (a distance between a pair of atoms causing ring opening was within 5 Å) were selected.

For the cyclic peptide in which 6 glycines were bonded to form a ring, energy minimization was performed on 14,734 conformations.

For the cyclic peptide in which 8 glycines were bonded to form a ring, energy minimization was performed on 1,573,436 conformations.

Note that, in case of cyclic peptide in which 10 glycine are bonded to form a ring, it is expected that a calculation time thereof is 4⁴ times the calculation time of cyclic peptide in which 8 glycines are bonded to form a ring. This is from the following reason.

As the number of glycine residue increases by 1, the number of rotatable bonds increases by 2. Since a structure is changed by 4 angles every 90 degrees per bond, the number of conformations to be generated increases 4²-fold.

The time spent on energy minimization according to the method of Comparative Example 4 is presented in Table 2.

The calculator used for the calculation of energy minimization was the same as the calculator used in Example 4.

**Table 2**

| | Comparative Example 4 (time) | Example 4 (time) |
|---|---|---|
| 6 residues | 1.5 | 0.5 |
| 8 residues | 273 | 5.5 |
| 10 residues | 69,900 | 105 |

In Table 2, the number of the residues represents the number of glycine residues in the cyclic peptide.

### Reference Signs List

- 10:: device for calculating stable conformation
- 11:: CPU
- 12:: memory
- 13:: memory unit
- 14:: display unit
- 15:: input unit
- 16:: output unit
- 17:: I/O interface unit
- 18:: system bus
- 19:: network interface unit
- 20:: network interface unit
- 30:: computer
- 40:: computer

## Claims

1. A method for calculating stable conformations, the method comprising:
converting stable conformation data of an (n - a)-membered cyclic compound [with the proviso that a is a positive integer and (n - a) ≥ 3] into conformation data of an n-membered cyclic compound (with the proviso that n is an integer of 4 or greater) using data related to an atomic group including atoms in the number of at least a; and
minimizing energy of the n-membered cyclic compound using the conformation data of the n-membered cyclic compound,
wherein the method is a method for calculating stable conformations of the n-membered cyclic compound using a computer.

2. The method for calculating stable conformations according to claim 1,
wherein the converting includes converting data related to a bond constituting a ring of the (n - a)-membered cyclic compound into data related to bonds, the number of which is a + 1, generated between two atoms constituting the bond and the atoms in the atomic group where the number of the atoms is a.

3. The method for calculating stable conformations according to claim 1 or 2,
wherein the converting includes obtaining a plurality of sets of the conformation data of a single molecule using the data related to the atomic group as a plurality of coordinate patterns.

4. The method for calculating stable conformations according to any one of claims 1 to 3,
wherein the number of atoms in the atomic group is from 1 to 100.

5. The method for calculating stable conformation according to any one of claims 1 to 4,
wherein the number a is from 1 to 10.

6. A program for causing a computer to execute a method for calculating stable conformations of an n-membered cyclic compound, the method comprising:
converting stable conformation data of an (n - a)-membered cyclic compound [with the proviso that a is a positive integer and (n - a) ≥ 3] into conformation data of the n-membered cyclic compound (with the proviso that n is an integer of 4 or greater) using data related to an atomic group including atoms in the number of at least a; and
minimizing energy of the n-membered cyclic compound using the conformation data of the n-membered cyclic compound.

7. The program according to claim 6,
wherein the converting includes converting data related to a bond constituting a ring of the (n - a)-membered cyclic compound into data related to bonds, the number of which is a + 1, generated between two atoms constituting the bond and the atoms in the atomic group where the number of the atoms is a.

8. The program according to claim 6 or 7,
wherein the converting includes obtaining a plurality of sets of the conformation data of a single molecule using the data related to the atomic group as a plurality of coordinate patterns.

9. The program according to any one of claims 6 to 8,
wherein the number of atoms in the atomic group is from 1 to 100.

10. The program according to any one of claims 6 to 9,
wherein the number a is from 1 to 10.

11. A device for calculating stable conformations, the device comprising:
a converting unit configured to convert stable conformation data of an (n - a)-membered cyclic compound [with the proviso that a is a positive integer and (n - a) ≥ 3] into conformation data of an n-membered cyclic compound (with the proviso that n is an integer of 4 or greater) using data related to an atomic group including atoms in the number of at least a; and
a minimizing unit configured to minimize energy of the n-membered cyclic compound using the conformation data of the n-membered cyclic compound,
wherein the device is a device for calculating stable conformations of the n-membered cyclic compound.

12. The device according to claim 11,
wherein the converting unit is configured to convert data related to a bond constituting a ring of the (n - a)-membered cyclic compound into data related to bonds, the number of which is a + 1, generated between two atoms constituting the bond and the atoms in the atomic group where the number of the atoms is a.

13. The device according to claim 11 or 12,
wherein the converting unit is configured to obtain a plurality of sets of the conformation data of a single molecule using the data related to the atomic group as a plurality of coordinate patterns.

14. The device according to any one of claims 11 to 13,
wherein the number of atoms in the atomic group is from 1 to 100.

15. The device according to any one of claims 11 to 14,
wherein the number a is from 1 to 10.
